# EUROPEAN PATENT SPECIFICATION

(11) **EP 1 142 873 B1**
(45) Date of publication and mention of the grant of the patent: **21.04.2004**
(21) Application number: 01111213.3
(22) Date of filing: 24.11.1998
(51) Int. Cl.: C07D 209/88, C07D 209/08

(54) **Process for preparing 1-[9'H-carbazol-4'-yloxy]-3-[ 2''-(2'''- methoxy -phenoxy)-ethyl -amino]-propan-2-ol[carvedilol]**
Verfahren zur Herstellung von 1-(9'H-Carbazol-4'-yloxy)-3-(2''-(2'''-methoxy-phenoxy)-ethylamino)-propan-2-ol ( Carvediol)
Procédé pour la préparation de 1-(9'H-carbazol-4'-yloxy)-3-(2''-(2'''-méthoxy-phénoxy)-éthylamino)-propan-2-ol (carvediol)

(30) Priority: 24.11.1997 HU 9702209; 01.10.1998 HU 9802180
(43) Date of publication of application: 10.10.2001
(62) Divisional of application: 98122114.6
(73) Proprietor: EGIS GYOGYSZERGYAR RT., 1106 Budapest (HU)
(72) Inventor: Ratkai, Zoltan, 1101 Budapest (HU); Barkoczy, Jozsef, Dr., 1016 Budapest (HU); Simig, Gyula, Dr., 1126 Budapest (HU); Gregor, Tamas, Dr., Csömör (HU); Vereczkey, Györgyi Donath, 1036 Budapest (HU); Németh, Norbert, 9400 Sopron (HU); Nagy, Kalman, Dr., 1025 Budapest (HU); Cselenyak, Judit, 1124 Budapest (HU); Szabo, Tibor, 1144 Budapest (HU); Balazs, Laszlo, 1088 Budapest (HU); Doman, Imre, 1035 Budapest (HU); Greff, Zoltan, Dr., 1028 Budapest (HU); Nagy, Péter Kotay, Dr., 2600 Vac (HU); Seres, Péter, Dr., 1153 Budapest (HU)
(74) Representative: Beszédes, Stephan G., Dr.

(56) References cited:
- DE-A- 2 815 926

## Description

The present invention is concerned with a new process for preparing 1-[9'H-carbazol-4'-yloxy]-3--[{2''-(2'''-<methoxy>-phenoxy)-ethyl}-amino]-propan-2-ol [carvedilol] of formula 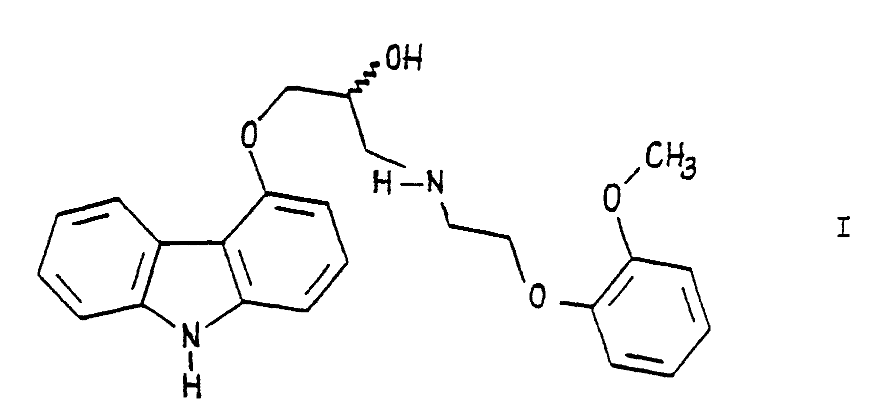 as well as acid addition salts of this compound.

This compound is known under the INN name carvedilol and is used as a drug having antihypertensive, beta-adrenergic blocking and vasodilating activity. The chemical name thereof is as beforesaid, 1-[9'H-carbazol-4'-yloxy]-3-[{2''-(2'''--<methoxy>-phenoxy)-ethyl}-amino]-propan-2-ol.

The preparation of 1-[9'H-carbazol-4'-yloxy]-3-[{2''-(2'''--<methoxy>-phenoxy)-ethyl}-amino]-propan-2-ol [carvedilol] has been known from DE-OS No. 28 15 926, while the preparation of the R and S enantiomers has been described in DE-OS No. 33 19 027. According to the known process, 4-(oxiranylmethoxy)-- 9H-carbazole of formula 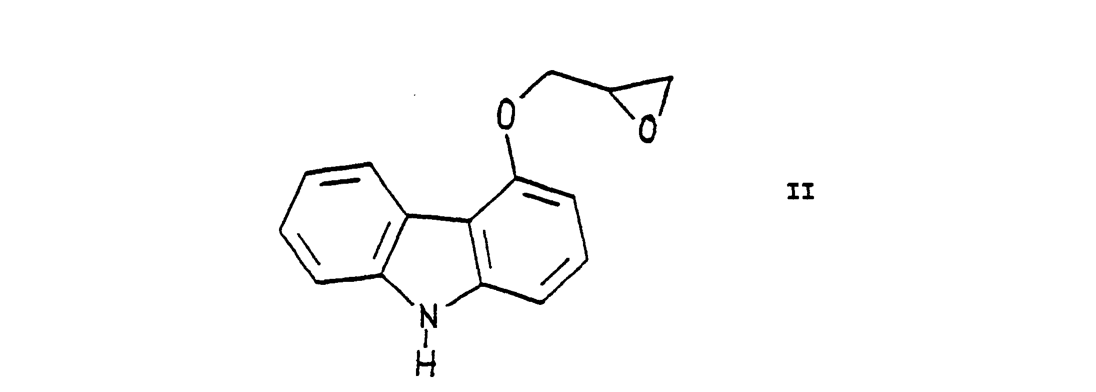 or the R or S enantiomer thereof is reacted with 2-[2'-- (methoxy)-phenoxy]-ethylamine of formula 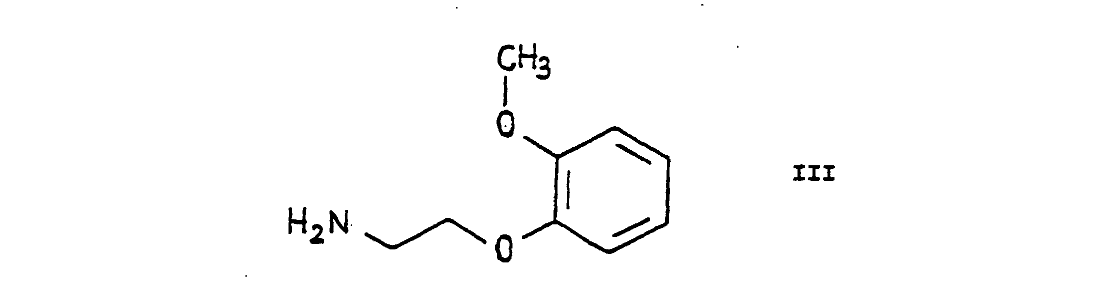 to produce the compound 1-[9'H-carbazol-4'-yloxy]-3-[{2''-- (2'''-<methoxy>-phenoxy)-ethyl}-amino]-propan-2-ol [carvedilol] of formula I in a yield of 39 to 42%. The drawback of the known process consists in the fact that parallel with the formation of 1-[9'H-carbazol-4'-yloxy]-3-[{2''-(2'''--<methoxy>-phenoxy)-ethyl}-amino]-propan-2-ol [carvedilol] also a bis compound of formula 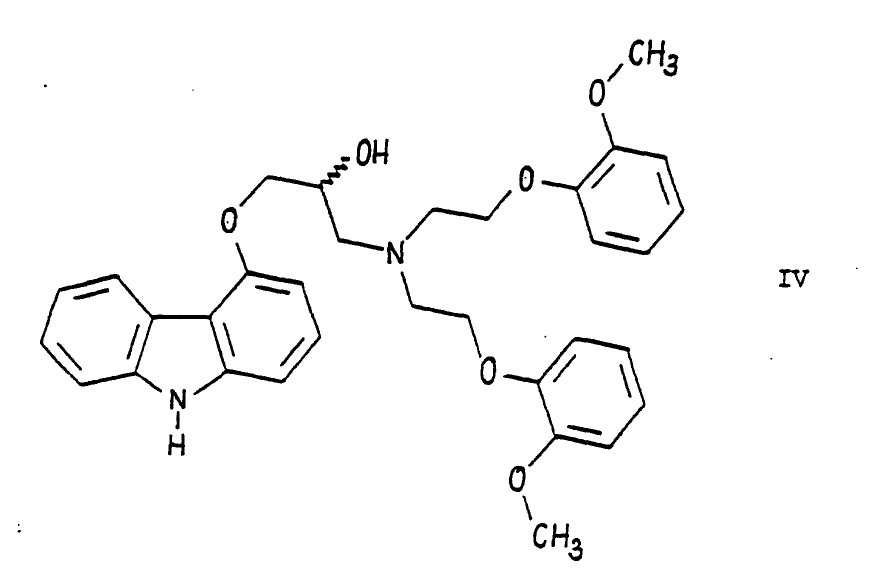 forms through the reaction of 2 molar equivalents of 4-(oxiranylmethoxy)-9-H-carbazole of formula II and 1 molar equivalent of 2-[2'-(methoxy)-phenoxy]-ethylamine of formula III. This side-reaction can never be avoided, and the bis compound of formula IV is formed in an amount that is commensurable with that of 1-[9'H-carbazol-4'-yloxy]-3-[{2''-- (2'''-<methoxy>-phenoxy)-ethyl}-amino]-propan-2-ol [carvedilol]. Consequently, the known process is uneconomical.

Also further possibilities for the preparation of [carvedilol] have been described in DE-OS No. 28 15 926. According to these processes, 4-[3'-(amino)-2'-(hydroxy)-- propoxy]-9H-carbazole can be reacted with
a) 2-[2'-(methoxy)-phenoxy]-ethylhalide or -sulfonate;
b) 2-[2'-(methoxy)-phenoxy]-acetaldehyde followed by catalytic hydrogenation;
c) 2-[2'-(methoxy)-phenoxy]-acetylchloride, followed by reducing the formed acid amide with a complex metal hydride.

None of the processes a) to c) is suitable for the economical preparation of 1-[9'H-carbazol-4'-yloxy]-3-[{2''-- (2'''-<methoxy>-phenoxy)-ethyl}-amino]-propan-2-ol [carvedilol]. In case of each process, a purification step by column chromatography is required for the separation of the product, and the preparation of the starting compound is uneconomical. In process a), the bis compound of formula IV forms, too. In case of process b), a yield of 41%, while in case of process c), a yield of 24% can be obtained, at the most. In process c), the use of a complex metal hydride, practically lithium aluminium hydride, is also a drawback since this reaction is accompanied by an enhanced fire hazard. The latter reaction requires absolute conditions, i.e. even traces of water should be avoided.

In principle, the formation of the bis compound of formula IV can be avoided by using, instead of the primary amine 2-[2'-- (methoxy)-phenoxy]-ethylamine of formula III, a secondary amine which is a derivate of the primary amine 2-[2'-(methoxy)--phenoxy]-ethylamine of formula III containing a protective group. Such a secondary amine is, for example, the N-[2-(2'--<methoxy>-phenoxy)-ethyl]-benzylamine of formula

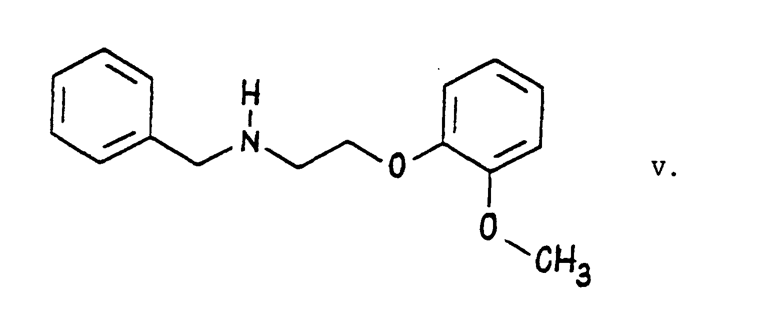

In Example 5 of DE-OS No. 28 15 926, the 4-(oxiranylmethoxy)-- 9H-carbazole of formula II is reacted with the secondary amine N-[2-(2'-<methoxy>-phenoxy)-ethyl]-benzylamine of formula V to yield the 1-[N-{benzyl}-2'-({2''-<methoxy>-phenoxy)-ethyl}--amino]-3-[9'''H-carbazol-4'''-yloxy]-propan-2-ol [benzyl--carvedilol] of the formula 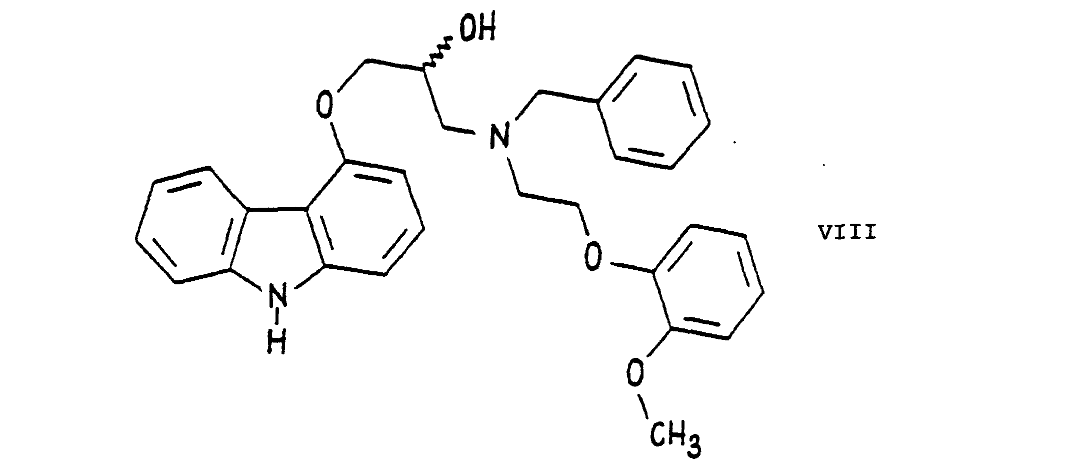 which could be separated only after a purification step by column chromatography. This procedure is uneconomical for manufacturing on an industrial scale. The reaction of the compounds of the formulae II and V was carried out in ethylene glycol dimethyl ether.

Example 5 of DE-OS No. 28 15 926 was reproduced on a fourfold scale. As shown by Example for Comparison No. I, only a yield of 60% could be achieved, and a contaminated product was obtained which has a melting point of 92°C significantly lower than the value indicated in Example 5 of DE-OS No. 28 15 926 (97 to 99°C) and also lower than the values obtained in Examples 1 (93 to 95°C) and 4 (94 to 96°C) of the present application. Without purification by column chromatography, it was failed to crystallize the product, thus, this purification procedure cannot be avoided. This is a considerable disadvantage in case of industrial application. Since the enlargement of scale of the above known process is accompanied by the reduction of both yield and quality of the product, said process is not suitable as an industrial one.

The process indicated in Example 5 of DE-OS No. 28 15 926 was modified by using, instead of ethylene glycol dimethyl ether, ethyl acetate or dioxan as shown by Examples for Comparison Nos. II and III. Even after a reaction period of 28 hours, about half of the starting compound remained unreacted in dioxan, and practically no reaction has taken place in ethyl acetate.

The problem underlying to the invention is to provide for a new economical process for preparing 1-[9'H-carbazol-4'-yloxy]-3-[{2''-(2'''-<methoxy>-phenoxy)--ethyl}-amino]-propan-2-ol [carvedilol] by which by overcoming the above disadvantages of the known processes this compound can be obtained with high yields and in a simple way with drastically reduced formation of byproducts and thus without the necessity of purification steps, which process also can be carried out on large scale industrially.

Surprisingly this has been achieved by the present invention.

The subject matter of the invention is a process for preparing 1-[9'H-carbazol-4'-yloxy]-3-[{2''-(2'''-<methoxy>--phenoxy)-ethyl}-amino]-propan-2-ol [carvedilol] of formula 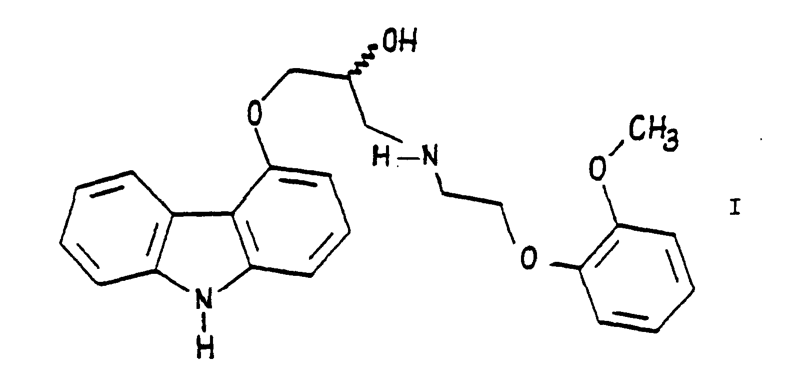 as well as acid addition salts of this compound, which is characterized in that the secondary amine N-[2-(2'-<methoxy>-- phenoxy)-ethyl]-benzylamine of formula 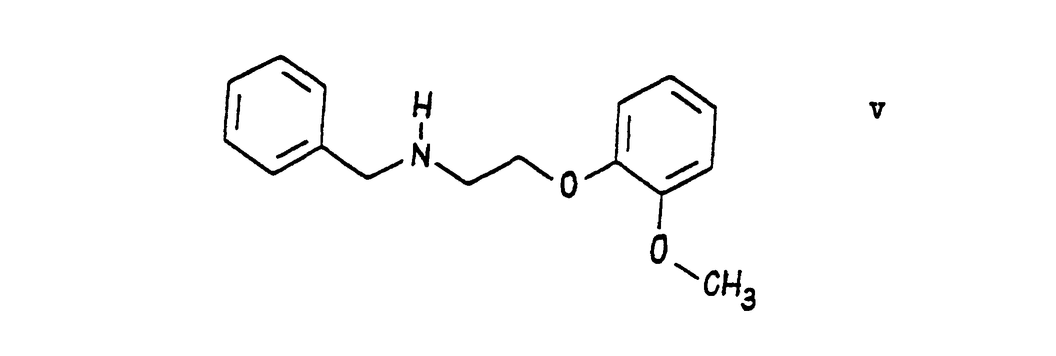 is reacted with epichlorohydrin in a manner known per se, the thus formed chloro compound 1-[N-{benzyl}-2'-({2''--<methoxy>-phenoxy)-ethyl}-amino]-3-[chloro]-- propan-2-ol of formula 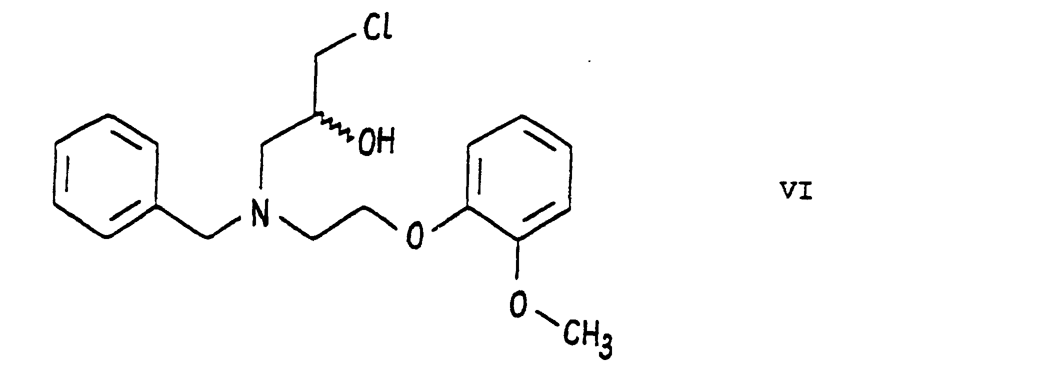 is reacted with 4-(hydroxy)-9H-carbazole of formula 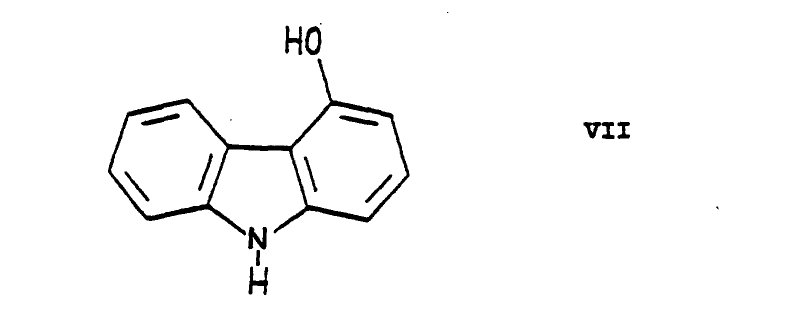 and the thus formed 1-[N-{benzyl}-2'-({2''-<methoxy>-- phenoxy)-ethyl}-amino]-3-[9'''H-carbazol-4'''-- yloxy]-propan-2-ol [benzyl-carvedilol] of formula VIII is debenzylated by catalytic hydrogenation
and, in a manner known per se, if desired, the 1-[9'H-carbazol--4'-yloxy]-3--[{2''-(2'''-<methoxy>-phenoxy)-ethyl}-amino]-- propan-2-ol thus obtained is reacted with inorganic or organic acids to yield acid addition salts thereof or, if desired, liberating the free 1-[9'H-carbazol-4',-yloxy]-3-[{2''-(2'''--<methoxy>-phenoxy)-ethyl}-amino]-propan-2-ol base [carvedilol] of formula I from acid addition salts thereof and, if desired, converting the free 1-[9'H-carbazol-4'-yloxy]-3-[{2''-(2'''--<methoxy>-phenoxy)-ethyl}-amino]-propan-2-ol base [carvedilol] into other acid addition salts and/or, if desired, separating the enantiomers.

The secondary amine N-[2-(2'-<methoxy>-phenoxy)-ethyl]-benzylamine of formula V can be employed as an oil, i.e. in a form without crystal water, but preferably in a form containing crystal water.

In the process according to the invention, the reaction of the secondary amine N-[2-(2'-<methoxy>-- phenoxy)-ethyl]-benzylamine of formula V with the epichlorohydrin is carried out in the absence or presence of a solvent, advantageously at a temperature of 0 to 150°C. The solvent can be an organic solvent of protic, dipolar aprotic or apolar character. It is preferred to perform the reaction in the absence of any solvent; in this case suitably an excess of epichlorohydrin is used and the reaction is carried out at a temperature of 30 to 80°C.

Suitably the chloro compound 1-[N-{benzyl}-2'-({2''--<methoxy>-phenoxy)-ethyl}-amino]-3-[chloro]-propan-2-ol of formula VI is reacted with the 4-(hydroxy)-9H-carbazole of formula VII in an organic solvent of dipolar aprotic or apolar character at a temperature of 0 to 150°C. A preferred solvent is acetonitrile, and the reaction is performed suitably at the boiling point of the reaction mixture.

In the process according to the invention, the formed 1-[N-{benzyl}-2'-({2''-<methoxy>-- phenoxy)-ethyl}-amino]-3-[9'''H-carbazol-4'''-yloxy]--propan-2-ol [benzyl-carvedilol] of formula VIII is either separated and then debenzylated, or preferably it is not separated from the reaction mixture in which it was prepared before the debenzylation reaction. The debenzylation by catalytic hydrogenation can be carried out in a manner known per se in connection with the removal of a benzyl group.

Preferably as a catalyst palladium on carbon is used. The catalyst can be used several times, without regeneration procedure, for the debenzylation of the 1-[N-{benzyl}-2'-({2''--<methoxy>-phenoxy)-ethyl}-amino]-3-[9'''H-carbazol-4'''-- yloxy]-propan-2-ol [benzyl-carvedilol] of formula VIII. Preferably the hydrogenation is carried out using hydrazine hydrate.

The secondary amine N-[2-(2'-<methoxy>-phenoxy)-ethyl]--benzylamine of formula V can have been prepared by the method of Augstein (J. Med. Chem., 8 [1965], 356 to 367) or in the manner given in the description.

1-[9'H-carbazol-4'-yloxy]-3-[{2''-(2'''-<methoxy>-phenoxy)--ethyl}-amino]-propan-2-ol [carvedilol] of very high purity is produced in a yield of about 80% by the process of the invention. In the process no bis compound of formula IV forms.

Also the 1-[N-{benzyl}-2'-({2''-<methoxy>-phenoxy)-ethyl}--amino]-3-[9'''H-carbazol-4'''-yloxy]-propan-2-ol [benzyl--carvedilol] of formula VIII is obtained in a very pure state, and consequently no separation is required, and it can be debenzylated in the reaction mixture in which it was formed.

On the basis of the above characteristics, the process of the invention can be carried out simply and economically.

The process according to the invention is surprising in that by it the side-reaction forming the bis compound of formula IV is avoided. Thereby the product of suitable purity without purification by chromatography of the 1-[N-{benzyl}-2'-({2''-<methoxy>-phenoxy)-ethyl}-amino]-3--[9'''H-carbazol-4'''-yloxy]-propan-2-ol [benzyl-carvedilol] of formula VIII can be prepared in an economic way.

The invention is further elucidated by means of the following Examples.

### Example 1

a) 1-[N-{Benzyl}-2'-({2''-<methoxy>-phenoxy)-ethyl}--amino]-3-[chloro]-propan-2-ol
   10.3 g (40 mmoles) of N-[2-(2'-<methoxy>-phenoxy)--ethyl]-benzylamine are dissolved in 16 ml of epichlorohydrin, and the solution obtained is stirred at 55 to 60°C for 3 hours. The reaction mixture is evaporated under reduced pressure, 20 ml of toluene are added, evaporated and the procedure is repeated.
   Thus, 13.8 g (99%) of the title compound 1-[N-{benzyl}-2'-({2''-<methoxy>-phenoxy)-ethyl}--amino]-3-[chloro]-propan-2-ol are obtained as an oily evaporation residue.
b) 1-[N-{Benzyl}-2'-({2''-<methoxy>-phenoxy)-ethyl}--amino]-3-[9'''H-earbazol-4'''-yloxy]-propan-2-ol
   3.5 g (10 mmoles) of 1-[N-{benzyl}-2'-({2''--<methoxy>-phenoxy)-ethyl}-amino]-3-[chloro]-propan--2-ol, prepared as described under a), and 0.92 g (5 mmoles) of 4-(hydroxy)-9H-carbazole are dissolved in 25 ml of dioxan. To the solution obtained, 0.69 g (5.0 mmoles) of potassium carbonate are added, and the reaction mixture is boiled for 28 hours under stirring, then cooled to 25°C. The inorganic salt is filtered off, washed with dioxan and the filtrate is evaporated under reduced pressure. To the 4.6 g of evaporation residue 25 ml of isopropanol are added. The mixture is heated to 60°C, and allowed to cool under stirring. After 24 hours the crystals are filtered off and washed with diisopropyl ether.

Thus, 1.79 g (71.7%) of the title compound 1-[N-{benzyl}--2'-({2''-<methoxy>-phenoxy)-ethyl}-amino]-3-[9'''H-carbazol--4'''-yloxy]-propan-2-ol are obtained in the form of white crystals. M.p.: 93 to 95°C.

### Example 2

### 1-[9'H-Carbazol-4'-yloxy]-3-[{2''-(2'''-<methoxy>-- phenoxy)-ethyl}-amino]-propan-2-ol

Into a flask of 100 ml capacity 1.99 g (4 mmoles) of 1--[N-{benzyl}-2'-({2''-<methoxy>-phenoxy)-ethyl}-amino]-3--[9'''H-carbazol-4'''-yloxy]-propan-2-ol, prepared as described in Example 1 40 ml of ethanol, 4 ml of water and 1.0 g of wet palladium on carbon catalyst having a water content of 50.6% by weight and containing 16.2% by weight of palladium calculated on the dry catalyst are added. To the mixture cooled with cold water, 4 ml (80 mmoles) of 98% by weight hydrazine hydrate are added, drop by drop, at 5 to 10°C in 10 minutes, and the reaction mixture is stirred at 25°C for 2 hours. The catalyst is filtered off, washed twice using 20 ml of ethanol each time, and the filtrate is evaporated under reduced pressure. To the evaporation residue 50 ml of water and 50 ml of 1,2-dichloroethane are added, the mixture is stirred until dissolution, then the phases are separated, and the aqueous phase is further extracted twice using 50 ml of 1,2-dichloroethane each time. The organic phase is dried and evaporated under reduced pressure. The evaporation residue is heated to boiling with 9 ml of ethyl acetate, filtered through a folded paper filter, while hot, and the filtrate is allowed to cool under stirring. After the precipitation of the crystals, the mixture is stirred at 25°C for a further hour, the crystals are filtered off, and washed twice using 1 ml of cold ethyl acetate each time. The product is dried under a lamp emitting infrared radiation.

Thus, 1.33 g (81.6%) of the title compound 1-[9'H-carbazol-- 4'-yloxy]-3-[{2''-(2'''-<methoxy>-phenoxy)-ethyl}-amino]-- propan-2-ol are obtained as white crystals. M.p.: 114 to 116°C.

### Example 3

### 1-[9'H-Carbazol-4'-yloxy]-3-[{2''-(2'''-<methoxy>--phenoxy)-ethyl}-amino]-propan-2-ol

To 60 ml of ethanol, 1.1 g (2.2 mmoles) of 1-[N-{benzyl}--2'-({2''-<methoxy>-phenoxy)-ethyl}-amino]-3-[9'''H-carbazol--4'''-yloxy]-propan-2-ol, prepared as described in Example 1, and 0.5 g of wet palladium on carbon catalyst having a water content of 50.6% by weight and containing 16.2% by weight of palladium calculated on the dry catalyst are added. The mixture is vigorously stirred under a hydrogen atmosphere of 5 bar at room temperature. The reduction is finished in about 3 hours. The catalyst is filtered off, washed twice with ethanol using 20 ml of ethanol each time, and the filtrate is evaporated under reduced pressure. To the evaporation residue, 5 ml of ethyl acetate are added, the mixture is stirred at 25°C for 2 hours, the crystals obtained are filtered off, washed twice using 1 ml of cold ethyl acetate each time. The product is dried under a lamp emitting infrared radiation.

Thus, 0.69 g (76.6%) of the title compound 1-[9'H-carbazol--4'-yloxy]-3-[{2''-(2'''-<methoxy>-phenoxy)-ethyl}-amino]--propan-2-ol are obtained as white crystals. M.p.: 114 to 116°C.

### Example 4

### 1-[N-{Benzyl}-2'-({2''-<methoxy>-phenoxy)-ethyl}--amino]-3-[9'''H-carbazol-4'''-yloxy]-propan-2-ol

41.2 g (160 mmoles) of N-[2-(2'-<methoxy>-phenoxy)-ethyl]-- benzylamine are dissolved in 64 ml of epichlorohydrin, and the solution obtained is stirred at 55 to 60°C for 3.5 hours. Then, the reaction mixture is evaporated under reduced pressure, to the residue 20 ml of toluene are added, evaporated, and this procedure is repeated once more. To the 56.0 g of oily evaporation residue 300 ml of acetonitrile and 14.66 g (80 mmoles) of 4-(hydroxy)-9H-carbazole are added. To the solution obtained 13.45 g (160 mmoles) of sodium hydrogen carbonate are added, and the reaction mixture is stirred under boiling for 20 hours. The mixture is cooled to 25°C, the inorganic salt is filtered off, washed with acetonitrile. The filtrate is evaporated under reduced pressure. To the 68.6 g of evaporation residue, 160 ml of isopropanol are added. The mixture is heated to 60°C, and allowed to cool under stirring. In 3 hours a thick suspension is obtained which is cooled with ice-water, filtered, and washed with 100 ml of cold isopropanol and 100 ml of diisopropyl ether. The product is dried under a lamp emitting infrared radiation.

Thus, 31.80 g (80.0%) of the title compound 1-[N-{benzyl}--2'-({2''-<methoxy>-phenoxy)-ethyl}-amino]-3-[9'''H-carbazol--4'''-yloxy]-propan-2-ol are obtained as white crystals. M.p.: 94 to 96° C. Preparation of the starting compound:

### Preparation of N-[2-(2'-<methoxy>-phenoxy)-ethyl]--benzylamine of formula V

### N-[2- (2'-<methoxy>-phenoxy)-ethyl]--benzylaminedihydrate

To 131 ml (128.64 g, 1.2 moles) of benzylamine heated to 80°C 69.33 g (0.3 moles) of 1-[2'-(bromo)-ethoxy]-2-[methoxy]--benzene are added in 25 minutes. The rate of addition is adjusted to maintain the inner temperature of the reaction mixture at 95 to 105°C. After the addition, the mixture is stirred at an inner temperature of 95 to 100°C for 2 hours, the suspension obtained is cooled in ice-water to 25°C, then poured into 1 000 ml of 10% by weight hydrochloric acid cooled with ice-water in 5 minutes taking care that the inner temperature should not exceed 50°C. The solution obtained is cooled with ice-water. In 5 to 10 minutes, the hydrochloride of the product precipitates in the form of crystals. The crystal suspension is stirred at 5 to 10°C for half an hour, filtered, washed with water. The crude hydrochloride is recrystallized from 400 ml of water, filtered and washed with water.

Thus, 71.9 g (81.6%) of the hydrochloride of the title compound N-[2-(2'-<methoxy>-phenoxy)-ethyl]-benzylamine dihydrate are obtained. M.p.: 148 to 150°C.

The hydrochloride is suspended in 300 ml of water, the suspension is heated until the crystals dissolve, and, to the warm solution, 100 ml of 10% by weight aqueous sodium hydroxide solution are added, drop by drop. The solution is cooled with ice-water to a temperature of 5 to 10°C, the crystals are filtered, washed with 100 ml of cold water and dried on the air at room temperature.

Thus, 43.1 g (48.9%) of the title compound N-[2-(2'--<methoxy>-phenoxy)-ethyl]-benzylamine dihydrate are obtained. The product contains crystal water (C₁₆H₁₉NO₂.2H₂O). M.p.: 53 to 55°C.

### Example for Comparison No. I

### (Reproduction of Example 5 of DE-OS No. 28 15 926)

A mixture of 60.4 g of 4-(oxiranylmethoxy)-9H-carbazole, 64.8 g of N-[2-(2'-<methoxy>-phenoxy)-ethyl]-benzylamine and 200 ml of ethylene glycol dimethyl ether was boiled for 24 hours. On cooling the mixture, the product did not precipitate. The mixture was evaporated under reduced pressure, the residual oil could not be crystallized from ethanol, isopropanol and acetone, wherefore it was transferred to a column filled with silica gel. In the column chromatography 1,2-dichloromethane, a mixture containing 9 volumes of 1,2--dichloroethane and 1 volume of ethyl acetate, a mixture containing 7 volumes of 1,2-dichloroethane and 3 volumes of ethyl acetate, and, at last, ethyl acetate were used as the solvent. The fractions containing the product were combined and evaporated to yield 75 g (60%) of 1-[N-{benzyl}-2'-({2''--<methoxy>-phenoxy)-ethyl}-amino]-3-[9'''H-carbazol-4'''-- yloxy]-propan-2-ol <benzyl-carvedilol>. M.p.: 92°C.

### Example for Comparison No. II

A mixture of 1.92 g (8 mmoles) of 4-(oxiranylmethoxy)-9H--carbazole, 3.08 g (10.5 mmoles) of N-[2-(2'-<methoxy>-phenoxy)--ethyl]-benzylamine and 20 ml of ethyl acetate was boiled. The reaction was followed by means of HPLC. After 28 hours, the reaction mixture contained merely 3% by weight of 1-[N-{benzyl}-2'-({2''-<methoxy>-phenoxy)-ethyl}-amino]-3--[9'''H-carbazol-4'''-yloxy]-propan-2-ol [benzyl-carvedilol], practically the whole amount of the starting 4-(oxiranylmethoxy)-9H-carbazole remained unreacted. The characteristics of HPLC:
Column: LiChrospher 100RP-18 (5 µm), λ = 254 nm.
Acetonitrile: buffer solution = 1 : 1
flow velocity: 0.5 ml/min.
Buffer solution: 7.74 g of ammonium acetate and 10.5 ml of
acetic acid in 1 000 ml of aqueous solution.

### Retention times:

| | |
|---|---|
| N-[2-(2'-<methoxy>-phenoxy)-ethyl]-benzylamine | 2.0 min. |
| 4-(oxiranylmethoxy)-9H-carbazole | 4.6 min. |
| 1-[N-{benzyl}-2'-({2''-<methoxy>-phenoxy)--ethyl}-amino]-3-[9'''H-carbazol-4'''-yloxy]-- propan-2-ol [benzyl-carvedilol] | 14.1 min. |

### Example for Comparison No. III

The procedure of Example for Comparison No. II was repeated with the difference that 20 ml of dioxane were used as the solvent. After 28 hours of reaction, about 50% by weight of the amount of the starting 4-(oxiranylmethoxy)-9H-carbazole was still present as determined by HPLC.

## Claims

1. A process for preparing 1-[9'H-carbazol-4'-yloxy]-3--[{2''-(2'''-<methoxy>-phenoxy)-ethyl}-amino]-propan--2-ol [carvedilol] of formula 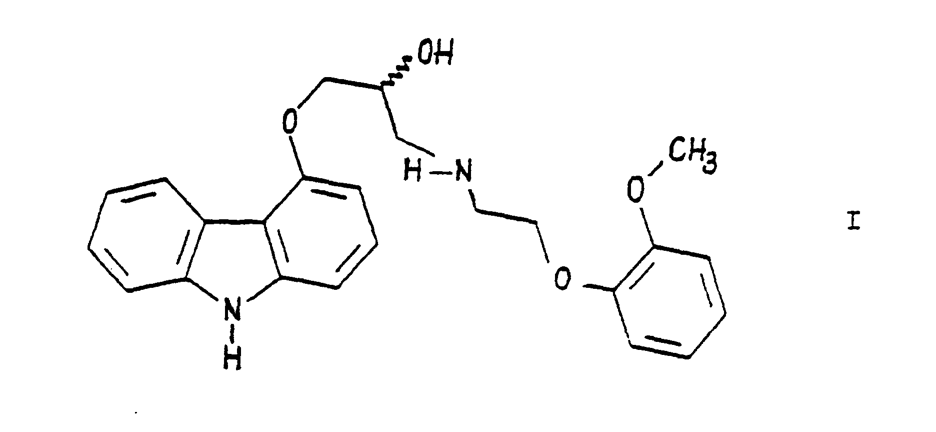 as well as acid addition salts of this compound, **characterized in that** the secondary amine N-[2-(2'--<methoxy>-phenoxy)-ethyl-benzylamine of formula 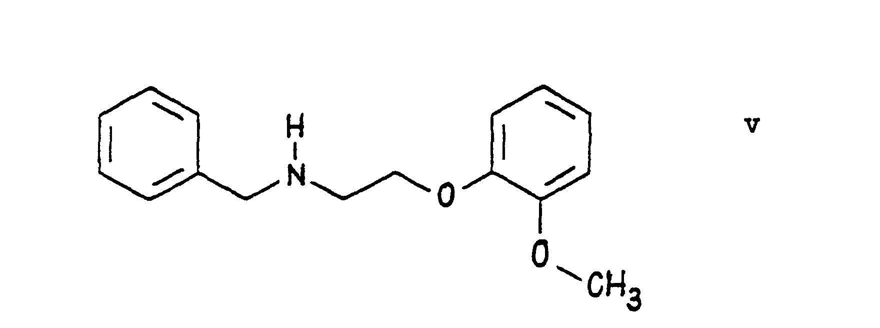 is reacted
with epichlorohydrin in a manner known per se, the thus formed chloro compound 1-[N-{benzyl}-2'-({2''-<methoxy>-phenoxy)-ethyl}-amino]-3--[chloro]-propan-2-ol of formula 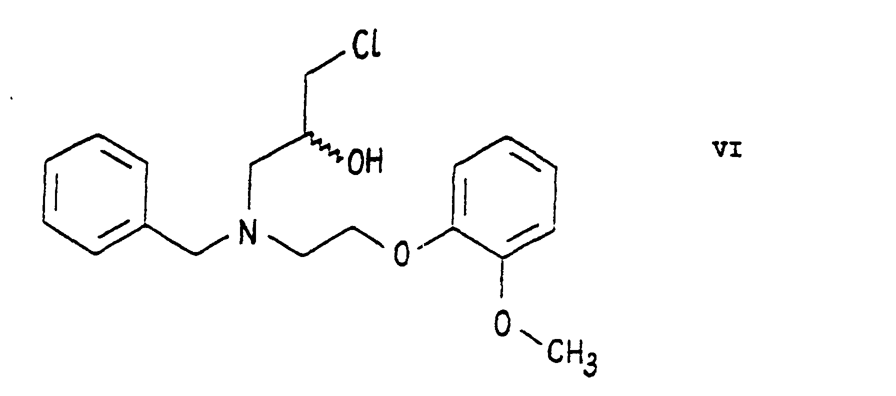 is reacted with 4-(hydroxy)-9H-carbazole of formula 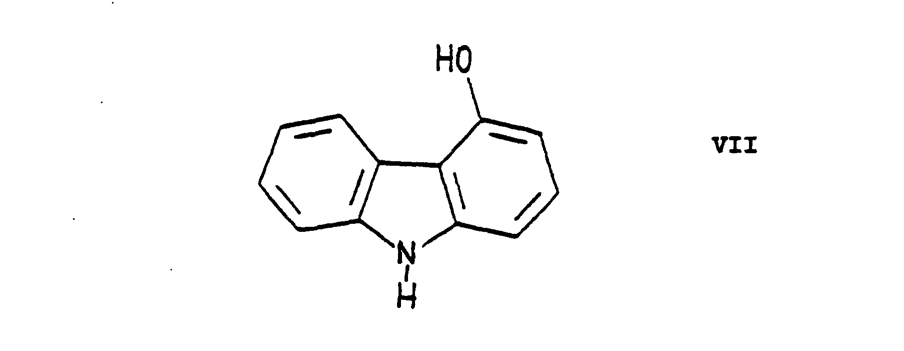 and the thus formed 1*-*[N-{benzyl}-2'-({2''--<methoxy>-phenoxy)-ethyl}-amino]-3-[9'''H--carbazol-4'''-yloxy]-propan-2-ol [benzyl-carvedilol] of formula VIII is debenzylated by catalytic hydrogenation and, in a manner known per se, if desired, the 1-[9'H-carbazol-4'-yloxy]-3-[{2''-(2'''--<methoxy>-phenoxy)-ethyl}-amino]-propan-2-ol thus obtained is reacted with inorganic or organic acids to yield acid addition salts thereof or, if desired, liberating the free 1-[9'H-carbazol-4'-yloxy]-3--[{2''-(2'''-<methoxy>-phenoxy)-ethyl}-amino]-propan--2-ol base [carvedilol] of formula I from acid addition salts thereof and, if desired, converting the free 1-[9'H-carbazol-4'-yloxy]-3-[{2''-(2'''--<methoxy>-phenoxy)-ethyl}-amino]-propan-2-ol base [carvedilol] into other acid addition salts and/or, if desired, separating the enantiomers.

2. A process according to claim 1, **characterized in that** the 1-[N-{benzyl}-2'-({2'''-<methoxy>-phenoxy)-ethyl}--amino]-3-[9'''H-carbazol-4'''-yloxy]-propan-2-ol [benzyl-carvedilol] of formula VIII is not separated from the reaction mixture in which it was prepared before the debenzylation reaction.

## Patentansprüche

1. Verfahren zur Herstellung von 1-[9'H-Carbazol-4'-yloxy]-3-[{2''-(2'''-<methoxy>phenoxy)-ethyl}-amino]-propan-2-ol [Carvedilol] der Formel 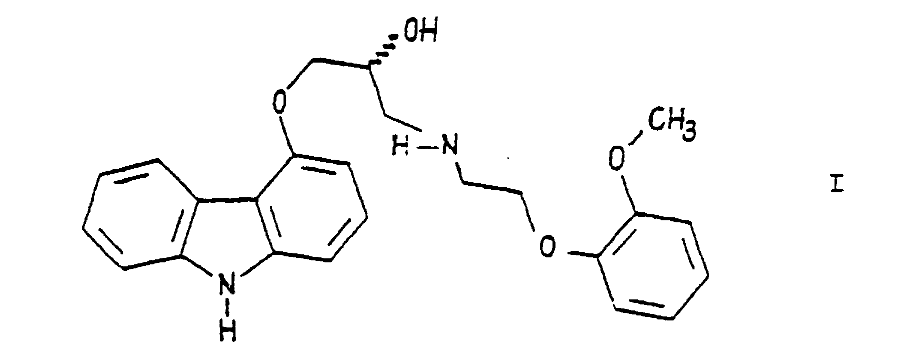 sowie von Säureadditionssalzen dieser Verbindung, **dadurch gekennzeichnet, dass** das sekundäre Amin N-[2-(2'-<methoxy>-phenoxy)-ethyl-benzylamin der Formel 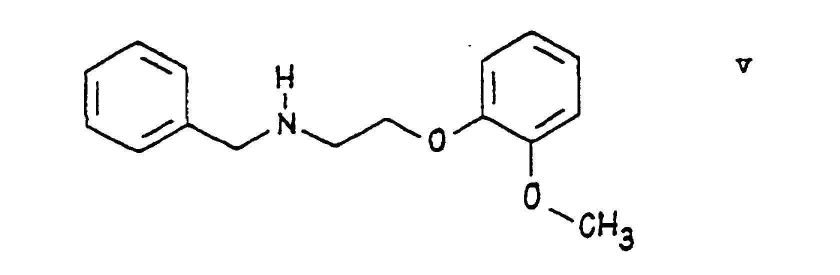 umgesetzt wird
mit Epichlorhydrin in einer an sich bekannten Weise, die so gebildete Chlorverbindung 1-[N-{Benzyl}-2'-({2''-<methoxy>-phenoxy)-ethyl}-amino]-3-[chlor]-propan-2-ol der Formel 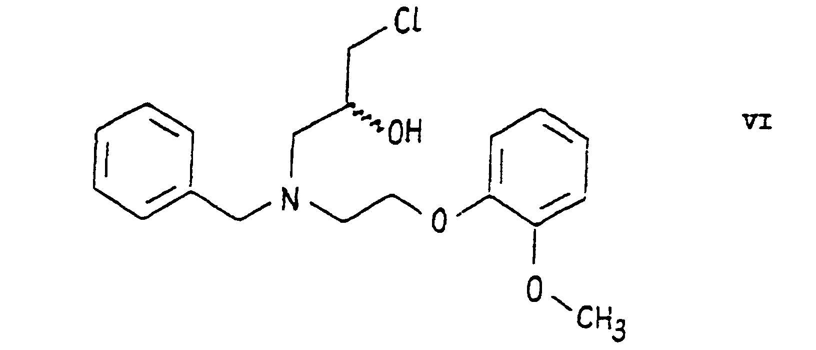 umgesetzt wird mit 4-(Hydroxy)-9H-carbazol der Formel 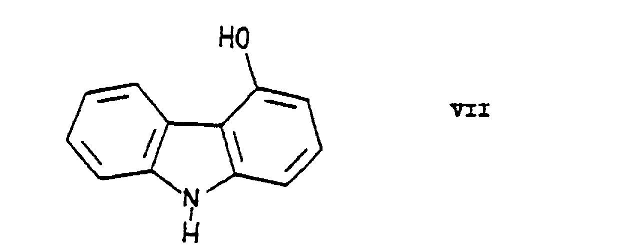 und das so gebildete 1-[N-{Benzyl}-2'-({2"-<methoxy>phenoxy)-ethyl}-amino]-3-[9'''H-carbazol-4'''-yloxy]-propan-2-ol [Benzyl-carvedilol] der Formel VIII debenzyliert wird durch katalytische Hydrierung und in einer an sich bekannten Weise auf Wunsch das so erhaltene 1-[9'H-Carbazol-4'-yloxy]-3-[{2''-(2'''-<methoxy>phenoxy)-ethyl}-amino]-propan-2-ol mit anorganischen oder organischen Säuren umgesetzt wird unter Erhalt von Säureadditionssalzen davon oder auf Wunsch die freie 1-[9'-H-Carbazol-4'-yloxy]-3-[{2"-(2'''-<methoxy>-phenoxy)-ethyl}-amino]-propan-2-ol-Base [Carvedilol] der Formel I von den Säureadditionssalzen davon befreit wird und auf Wunsch die freie 1-[9'-H-Carbazol-4'-yloxy]-3-[{2"-(2'''-<methoxy>-phenoxy)-ethyl}-amino]-propan-2-ol-Base [Carvedilol] in andere Säureadditionssalze umgewandelt wird und/oder auf Wunsch die Enantiomere abgetrennt werden.

2. Verfahren nach Anspruch 1, **dadurch gekennzeichnet, dass** das 1-[N-{Benzyl}-2'-({2"-<methoxy>phenoxy)-ethyl}-amino]-3-[9'''H-carbazol-4'''-yloxy]-propan-2-ol [Benzyl-carvedilol] der Formel VIII nicht von der Reaktionsmischung abgetrennt wird, in welcher es vor der Debenzylierungsreaktion hergestellt wurde.

## Revendications

1. Procédé pour la préparation du 1-[9'H-carbazol-4'-yloxy]-3-[{2''-(2'''-<méthoxy>-phénoxy)-éthyl}-amino]-propan-2-ol [carvédilol] de formule : 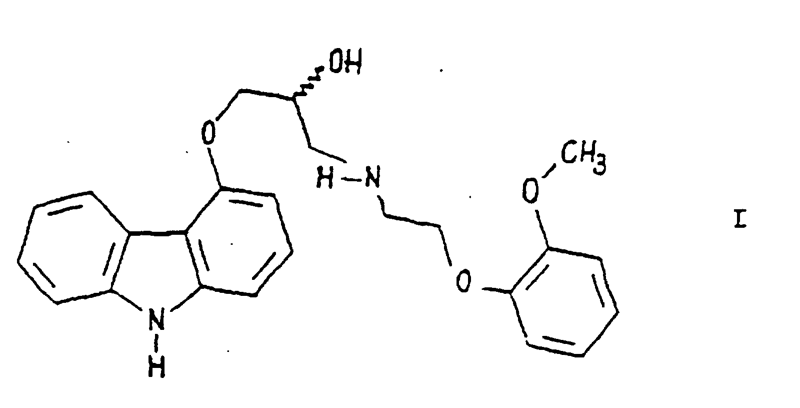 ainsi que de sels d'addition acide de ce composé, **caractérisé en ce que** l'amine secondaire N-[2-(2'-<méthoxy>-phénoxy)-éthyl-benzylamine de formule : 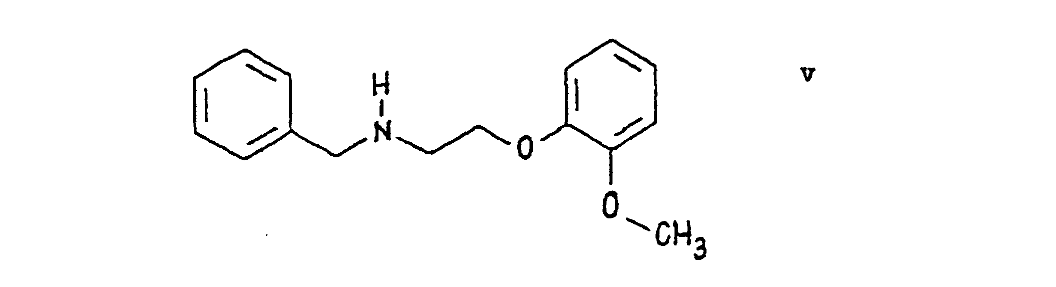 réagit avec de l'épichlorohydrine d'une manière connue en soi, le composé chloré ainsi formé 1-[N-{benzyl}-2'-({2''-<méthoxy>-phénoxy)-éthyl}-amino]-3-[chloro]-propan-2-ol de formule : 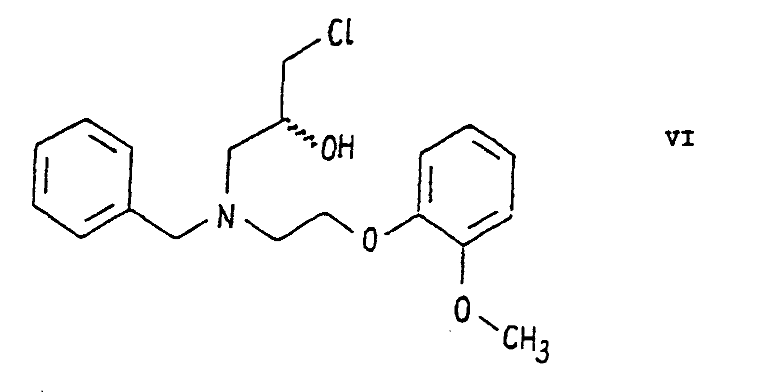 réagit avec le 4-(hydroxy)-9H-carbazole de formule : 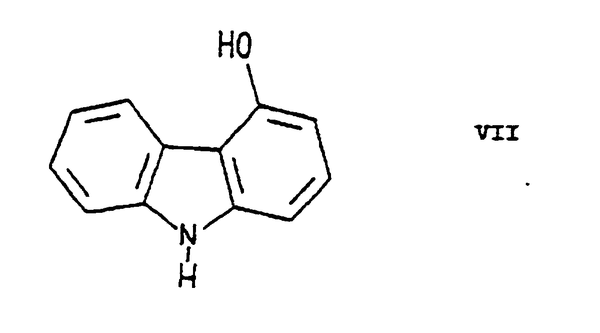 et le 1-[N-{benzyl}-2'-({2''-<méthoxy>-phénoxy)-éthyl}-amino]-3-[9'''H-carbazol-4'''-yloxy]-propan-2-ol [benzyl-carvédilol] de formule VIII ainsi formé est débenzylé par hydrogénation catalytique et d'une manière connue en soi, si souhaité, le 1-[9'H-carbazol-4'-yloxy]-3-[{2''-(2'''-<méthoxy>-phénoxy)-éthyl}-amino]-propan-2-ol ainsi obtenu réagit avec des acides inorganiques ou organiques pour donner des sels d'addition acide de celui-ci ou, si souhaité, en libérant la base libre 1-[9'H-carbazol-4'-yloxy]-3-[{2''-(2'''-<méthoxy>-phénoxy)-éthyl}-amino]-propan-2-ol [carvédilol] de formule I de ses sels d'addition acide et, si souhaité, en convertissant la base libre 1-[9'H-carbazol-4'-yloxy]-3-[{2''-(2'''-<méthoxy>-phénoxy)-éthyl}-amino]-propan-2-ol [carvédilol] en d'autres sels d'addition acide et/ou, si souhaité, en séparant les énantiomères.

2. Procédé selon la revendication 1, **caractérisé en ce que** le 1-[N-{benzyl}-2'-({2''-<méthoxy>-phénoxy)-éthyl}-amino]-3-[9'''H-carbazol-4'''-yloxy]-propan-2-ol [benzyl-carvédilol] de formule VIII n'est pas séparé du mélange réactionnel dans lequel il est préparé avant la réaction de débenzylation.
